# FASCICULE DE BREVET EUROPEEN

(11) **EP 0 403 945 B1**
(45) Date de publication et mention de la délivrance du brevet: **18.12.1996**
(21) Numéro de dépôt: 90111171.6
(22) Date de dépôt: 13.06.1990
(51) Int. Cl.: C07D 307/92, C07D 307/79, C07D 311/92, C07D 311/74

(54) **Carbinols cycloaliphatiques et leur utilisation à titre de produits de départ pour la préparation de dérivés furanniques**
Cycloaliphatische Carbinole und ihre Verwendung als Ausgangsverbindungen für die Herstellung von Furan-Derivaten
Cycloaliphatic carbinols and their use as starting materials for the preparation of furan derivatives

(30) Priorité: 19.06.1989 CH 2277/89
(43) Date de publication de la demande: 27.12.1990
(73) Titulaire: FIRMENICH SA, CH-1211 Genève 8 (CH)
(72) Inventeur: Schulte-Elte, Karl-Heinrich, CH-1213 Onex (CH); Snowden, Roger Leslie, F-74580 Viry (FR); Tarchini, Claudio, CH-1227 Carouge (CH); Baer, Béatrice, CH-1212 Grand-Lancy (CH); Vial, Christian, CH-1219 Le Lignon (CH)
(74) Mandataire: Salvaterra-Garcia, Maria de Lurdes

(56) Documents cités:
- EP-A- 0 165 458
- EP-A- 0 170 955
- DE-A- 2 848 095
- TETRAHEDRON LETTERS. vol. 27, n 38, 1986, OXFORD GB pages 4533-4536; M. E.Garst et al., page 4534, ligne 1-5
- TETRAHEDRON LETTERS. vol. 22, n 52, 1981, OXFORD GB pages 5259-5262; E.-J. BRUNKE et al.:" Kationische Cyclisierung von (Z)- und (E)-1,3-Dimethyl-2-(3- hexenyl)-2-cyclohexenol" * article en entier *
- TETRAHEDRON LETTERS. vol 29, n 20, 1988, OXFORD GB pages 2401-2404; V. EKOW AMOO et al.:"Synthesis of (S)-Manolide Diol and the Absolute Configuration of Natural Manolide " *page 2402, schéma I, formule 12; article en entier *
- CHEMICAL ABSTRACTS, vol. 114, n 15, 15 Avril 1991, Columbus, Ohio, US; abstract n 143747G, T. ORITANI et al. : "Preparation of Cyclic Terpenes " page 816 ; colonne 2; * abrégé * & JP-A-02258773 (KURARAY CO., Ltd.) 19 Octobre 1990

## Description

Le 3a alpha,5a bêta,9a alpha,9b bêta-dodécahydro-3a,6,6,9a-tétraméthylnaphto[2,1-b]furanne, composé de formule 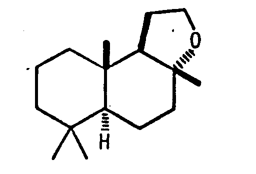 connu sous le nom d'AMBROX (marque enregistrée de Firmenich SA, Genève) est souvent accompagné dans des préparations commerciales de quantités variables de ses diastéréoisomères, épi-AMBROX et iso-AMBROX entre autres.

Depuis sa découverte [voir Helv. Chim. Acta 33, 1251 (1950)] nombreux ont été les procédés proposés pour son obtention. Ceux-ci sont généralement basés sur une réaction de dégradation oxydative de terpènes tel le (-)-sclaréol ou le (+)-manol, ou utilisent l'ambréine comme produit de départ [G. Ohloff dans Fragrance Chemistry, ed. Ernst T. Theimer, p. 545 et suiv., Academic Press (1982)]. Ces produits sont tous d'origine naturelle et par conséquent leur disponibilité et leur qualité dépendent de conditions climatiques variables et de facteurs socioéconomiques particuliers.

Extraits de la source naturelle avec des rendements modestes, ils sont donc accessibles à un prix qui rend leur utilisation à l'échelle industrielle peu économique.

Un procédé faisant état de la cyclisation de l'acide homofarnésique en norambréinolide, suivie de la réduction de la lactone obtenue et de la cyclisation du diol résultant pour fournir le composé furannique désiré a été décrit dans le brevet européen n° 107'857. La cyclisation de l'acide homofarnésique s'effectue selon le procédé précité au moyen de SnCl₄.

Une approche synthétique similaire avait été suggérée par A. Saito et al. [Chemistry Letters 757(1981)] qui avaient effectué la cyclisation de l'acide trans-bêta-monocyclohomofarnésique en utilisant le même SnCl₄ comme agent de cyclisation. Les deux documents cités reprennent par ailleurs les travaux réalisés par G. Lucius sur la cyclisation de l'acide homofarnésique [Angew. Chem. 68, 247 (1956); Arch. Pharm. 291, 57 (1958) et Chem. Ber. 93, 2663 (1960)].

Récemment, S. Neumann et H. Simon [Biol. Chem. Hoppe-Seiler 367(8), 723 (1986)] ont décrit la formation de 3a,6,6,9a-tétraméthyl-perhydro-naphto[2,1-b] furanne par une cylisation enzymatique de l'homofarnésol ou de l'éther d'homofarnésyl-(1,5,9-triméthyl-4,8-décadiényle). Un tel procédé cependant, vu la nature particulière des réactifs employés ainsi que des rendements et des taux de conversion observés, ne présente pour l'instant qu'un intérêt purement académique.

D'autres méthodes connues pour la préparation de l'AMBROX®, décrites par exemple dans EP-A2-170 955 et EP-A2-165 458, ont recours à la cyclisation d'un diol de formule 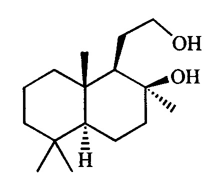 dans des conditions de réaction diverses et souvent mal adaptées à une exploitation industrielle. La synthèse de ce diol de départ est, par ailleurs, elle-même très complexe.

Finalement, la demande de brevet DE 28 48 95 A1 décrit un procédé pour la préparation d'un composé de formule 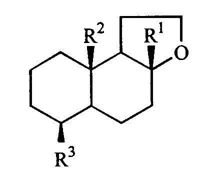 dans laquelle R¹ et R² représentent l'hydrogène ou un radical alkyle de C₁ à C₃ et R³ représente un radical alkyle de C₁ à C₃, par cyclisation d'un dérivé cyclohexanol de formule 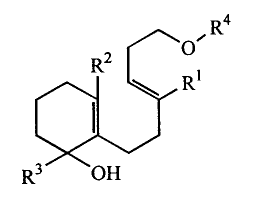 dans laquelle R⁴ représente l'hydrogène ou un groupe protecteur de la fonction alcool. Cette cyclisation mène à un intermédiaire de formule 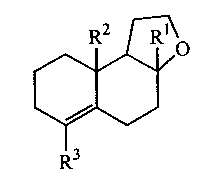 qui est ensuite hydrogéné pour fournir les produits désirés. Ce procédé à deux étapes n'est cependant pas adapté à la préparation de l'AMBROX®.

La contribution particulière que le composé offre à la parfumerie et la nécessité de pouvoir disposer d'un produit à un prix inférieur à celui pratiqué actuellement sur le marché nous ont incités à réexaminer ses méthodes de synthèse. La présente invention apporte une solution nouvelle et originale à ce problème.

L'invention a en effet pour objet un procédé pour la préparation d'éthers polycycliques de formule 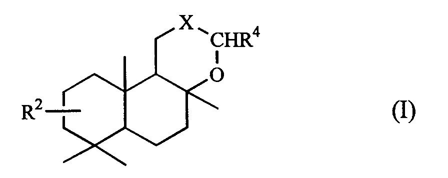 dans laquelle X représente un groupe ―(CH₂)ₙ―, l'indice n définit un nombre entier de valeur 0 ou 1, le symbole R² représente un atome d'hydrogène ou un radical alkyle inférieur de C₁ à C₃ et le symbole R⁴ représente un atome d'hydrogène ou un radical méthyle, caractérisé en ce qu'on cyclise à l'aide d'un agent acide un composé polyinsaturé de formule 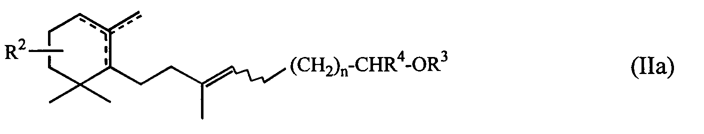 possédant une double liaison dans l'une des positions indiquées par les pointillés et dans laquelle les symboles n, R² et R⁴ sont définis comme à la formule (I), la ligne ondulée représente une liaison C-C de configuration cis ou trans et le symbole R³ représente un atome d'hydrogène ou un groupe protecteur de la fonction hydroxyle lié à l'atome d'oxygène et pouvant se dissocier de celui-ci dans les conditions de la réaction.

Comme indiqué ci-dessus, le symbole R³ représente un atome d'hydrogène ou un groupe protecteur de la fonction hydroxyle lié à l'atome d'oxygène et pouvant se dissocier de celui-ci dans les conditions de la réaction. Il s'agit dans ce cas de radicaux dont la liaison avec l'atome d'oxygène est labile dans le milieu acide dans lequel s'effectue la cyclisation. Des exemples de radicaux pouvant remplir cette fonction sont bien connus dans l'art. Il s'agit en particulier et à titre d'exemple de radicaux du type tétrahydropyrannyle, trialkylsilyle, tert-butyle ou acyle.

A titre d'agent acide de cyclisation, on peut employer un acide protonique minéral ou organique tel un acide carboxylique ou sulfonique ou encore un acide de type de Lewis. Parmi les acides minéraux, il convient de citer les acides phosphorique, sulfurique, perchlorique, les hétéropolyacides, par exemple le H₃[P(W₃O₁₀)₄] aqueux. On peut également utiliser des terres d'infusoires acides, des résines acides, telles le Dowex-50 [marque enregistrée de Dow Chemical CO. (USA)] ou l'Amberlyst IR-15 ou un oxyde d'alumine acide. Parmi les acides protoniques, on peut enfin mentionner les acides hydrohalogénés tel l'acide chlorhydrique, bromhydrique ou iodidrique.

A titre d'acide organique actif on peut employer l'acide trifluoroacétique ou l'acide acétique, en particulier un mélange d'acide acétique et sulfurique ou l'acide méthanesulfonique. Enfin, comme décrit plus haut, on peut utiliser un acide de Lewis. Des exemples particuliers de tels acides sont le trifluoroboroéthérate, le chlorure d'étain ou le tétrachlorure de titane.

La réaction de cyclisation s'effectue de préférence dans un milieu constitué par un solvant organique inerte. A cet effet, on peut opérer dans un solvant choisi parmi les hydrocarbures comme l'éther de pétrole, les hydrocarbures halogénés tels le chloroforme, le chlorure de méthylène ou le trichloroéthane, les hydrocarbures aromatiques, par exemple le benzène, le toluène, le chlorobenzène ou le méthoxybenzène, les éthers comme l'éther diméthylique, les esters comme l'acétate d'éthyle, ou un hydrocarbure nitré, par exemple le nitrométhane, le nitroéthane ou le nitroisopropane. Enfin des solvants comme le disulfure de carbone ou l'acétonitrile peuvent également être employés.

Quoique parfaitement reproductibles, les résultats obtenus à l'aide des différents réactifs acides cités, et les différents solvants mentionnés plus haut, diffèrent en fonction du choix particulier effectué par l'opérateur lors de la mise en oeuvre du procédé de l'invention. Il en va de même avec la température employée. La cyclisation peut en effet s'effectuer à une température comprise dans une gamme de valeurs allant de -60° à +25-30°C. Bien entendu, les rendements d'une part et le rapport isomérique des composés obtenus d'autre part varient en fonction du choix effectué par l'opérateur. Des modes opératoires préférentiels sont décrits dans les exemples qui suivent. Toute combinaison pouvant résulter d'un choix particulier d'agent acide, solvant, température et, bien entendu, produit de départ est fonction de la nature des produits finaux désirés.

La séparation des produits obtenus peut s'effectuer aisément par simple distillation.

Le procédé de l'invention présente, de par la simplicité du mode opératoire, un intérêt industriel majeur. Il s'agit en effet d'un procédé économique qui peut s'appliquer à la fabrication à grande échelle d'éthers polycycliques jusqu'ici difficilement accessibles par les méthodes synthétiques traditionnelles.

Le procédé de l'invention repose sur un type nouveau de cyclisation, aucun exemple de cyclisation d'un polyène du type sus-mentionné, comportant un groupe hydroxyle libre ou protégé en fin de chaîne, n'ayant été décrit, ni même suggéré dans l'art. D'autre part, il convient de souligner l'un des aspects majeurs de ce type de cyclisation; elle s'effectue de manière parfaitement stéréospécifique. Cette caractéristique du procédé de l'invention permet donc l'obtention des éthers polycycliques (I) dans l'une ou l'autre des formes isomériques désirées en fonction de la structure et de la configuration du produit de départ choisi. Ce résultat inattendu revêt un intérêt majeur dans la mesure où les propriétés des éthers obtenus, en particulier les propriétés organoleptiques dépendent de manière prononcée de leur configuration moléculaire.

Plus particulièrement, le procédé de l'invention permet l'obtention d'un mélange isomérique dont le contenu en l'isomère 3a alpha,5a bêta,9a alpha,9b bêta-dodécahydro-3a,6,6,9a-tétraméthyl-naphto[2,1-b]furanne est prépondérant. A cela s'ajoute le fait que la cyclisation, dans le cas du 4-méthyl-6-(2,6,6-triméthyl-cyclohex-2-ényl)-hex-3-énol pour fournir de l'AMBROX, s'opère avec formation préférentielle des composés trans-décaliniques. Au cours des différents essais effectués, la proportion des composés cis-décaliniques n'a pas dépassé en effet 15% en poids du mélange. Or de tels mélanges isomériques, dont la composition est nouvelle, possèdent les propriétés olfactives les plus prononcées et constituent par conséquent des ingrédients de choix pour le parfumeur dans son activité de création.

Cette prépondérance des composés de configuration trans-décalinique a également été observée lors de la cyclisation d'autres composés (IIa) comme il ressort des exemples présentés plus loin.

Quoique le procédé de l'invention trouve une application particulière dans la préparation d'AMBROX, sa mise en oeuvre peut permettre l'obtention d'éthers polycycliques variés ayant un intérêt pour la parfumerie et de ce fait il représente une méthode générale dont l'application peut apporter une solution originale pour la synthèse de composés odorants connus ou de structure inédite.

En ce qui concerne les produits de départ de formule (IIa), certains d'entre eux ont été décrits dans la littérature scientifique. C'est le cas notamment du 4-méthyl-6-(2,6,6-triméthyl-cyclohex-1-ényl)-hex-3-énol décrit dans Bull. Soc. Chim. France 1960, 1072 et Tetrah. Letters 1988, 29, 2401.

Les composés de formule (IIa) peuvent être obtenus à partir des aldéhydes de formule 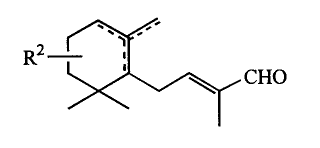 produits disponibles sur le marché ou pouvant être synthétisés à partir de composés connus.

Le procédé précité peut être illustré à l'aide du schéma suivant (n=0 dans la formule IIa) : 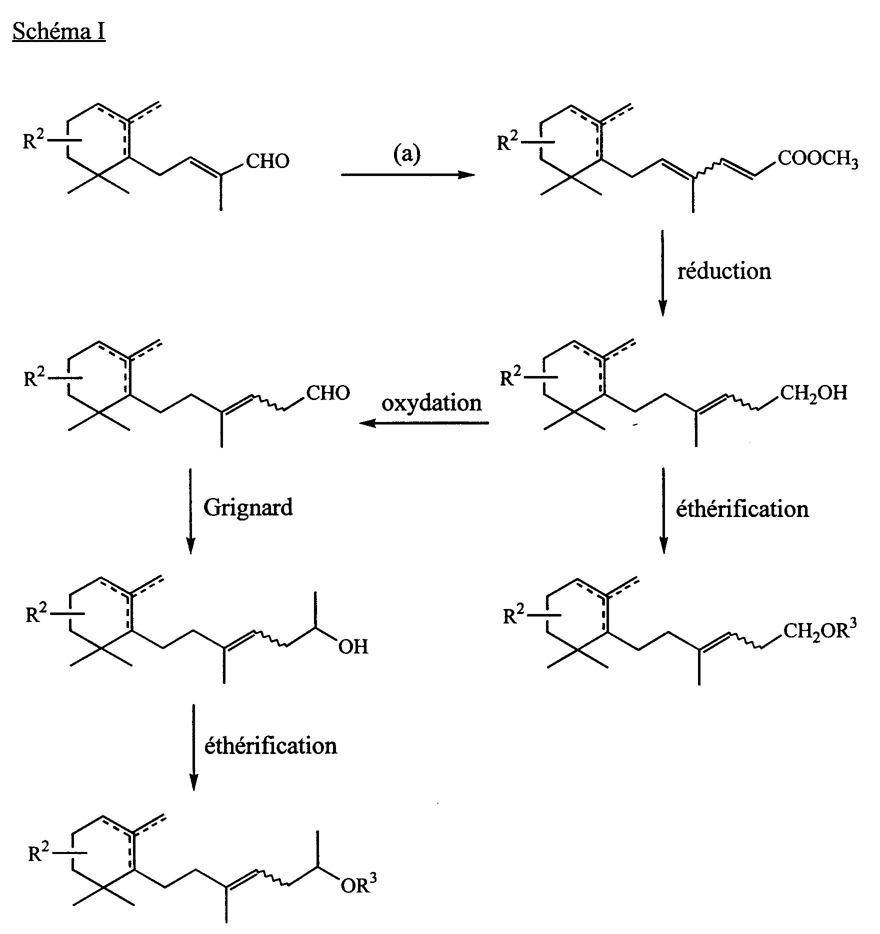
(a) réaction d'addition de dialkylphosphonoacétate d'alkyle (ex. : diméthylphosphonoacétate de méthyle) selon Homer-Emmons [voir : Chem. Rev. 1974, 74, 87]

A titre d'exemple des composés préparés a l'aide du procédé mentionné, il convient de citer les (E)- et (Z)-4-méthyl-6-[2,6,6-triméthyl-cyclohex-1(2)-enyl]-hex-3-énol et les (E)- et (Z)-4-méthyl-6-(2-méthylène-6,6-diméthyl-cyclohexyl)-hex-3-énol, composés définis par les formules suivantes:

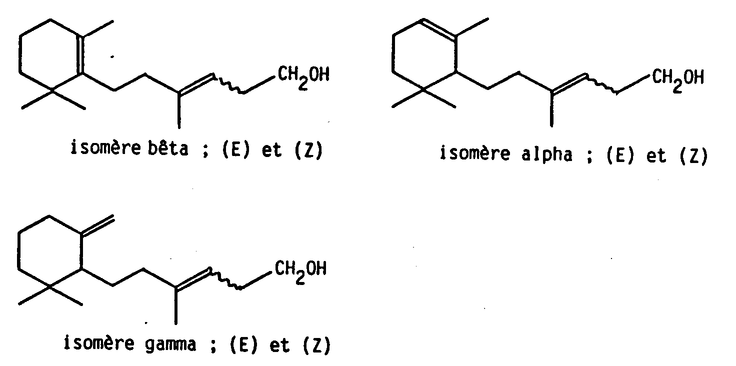

A titre d'agent réducteur du diène-ester obtenu dans l'étape (a) du procédé, on peut utiliser un réducteur métallique tel l'aluminohydrure de lithium, le diéthyl-aluminohydrure de sodium (OMH) ou encore le Vitride.

D'autres méthodes de préparation des composés (IIa) peuvent être utilisées et sont décrites en détail dans les exemples présentés ci-après.

Des cas particuliers de préparation des composés de formule (IIa) seront donnés dans les exemples qui suivent. Dans lesdits exemples, les températures sont indiquées en degrés centigrades et les abréviations ont le sens usuel dans l'art.

### Exemple 1

### Cyclisation acide de (E)- et (Z)-4-méthyl-6-(2,6,6-triméthyl-cyclohex-1-ényl)-hex-3-énol, (E)- et (Z)-4-méthyl-6-(2,6,6-triméthyl-cyclohex-2-ényl)-hex-3-énol, et (E)- et (Z)-4-méthyl-6-(2-méthylène-6,6-diméthyl-cyclohexyl)-hex-3-énol

La cyclisation du premier des composés mentionnés ci-dessus a été effectuée dans les conditions réactionnelles que voici. Une série d'essais a été conduite en maintenant constantes la nature et la concentration de l'agent acide. A cet effet, on a utilisé de l'acide sulfurique à 98% dans une proportion 1:1 en poids d'acide par rapport au produit de départ.
Dans la majorité des essais effectués, on a utilisé 2,4 g de produit de départ, lequel produit a été dissous dans 30 ml de solvant. Une seule exception, lors de l'utilisation de nitroéthane, une quantité inférieure de solvant a été employée.
En général, la température a été maintenue à -50°, excepté dans les cas où le solvant solidifiait à cette température ou lorsque le taux de conversion s'est révélé mauvais. Les résultats obtenus sont résumés dans le tableau ci-après.

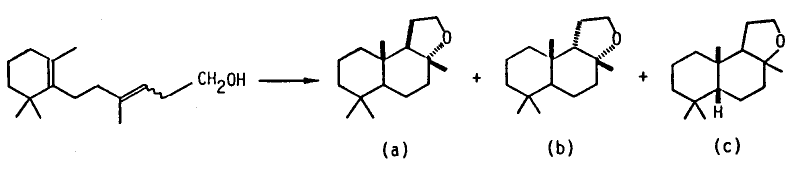

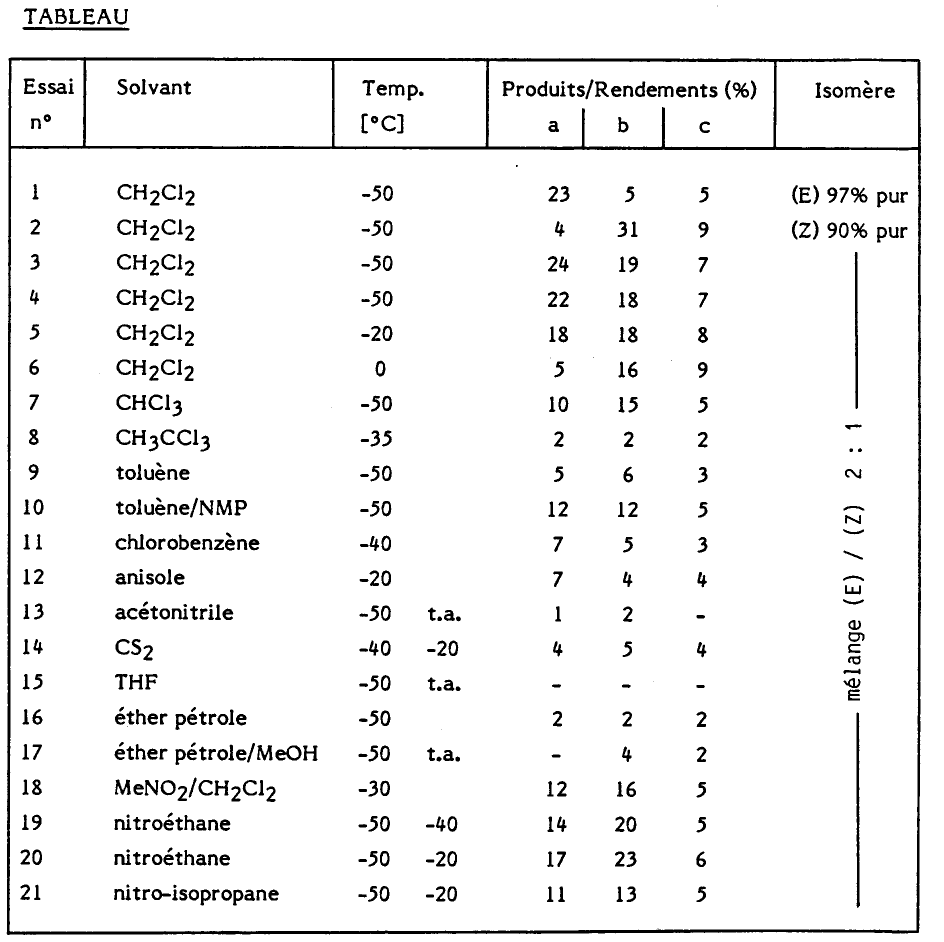

Dans tous les essais mentionnés, la réaction a été effectuée ainsi.
Une solution de l'alcool de départ (2,4 g; 8,9 mmole) dans 5 ml de solvant a été ajoutée goutte à goutte pendant 15 min à un mélange maintenu sous agitation d'acide sulfurique à 98% (2,4 g; 24 mmole) dans 25 ml de solvant à -50° sous azote. Après 3 h à -50°, le mélange a été neutralisé en le versant dans une solution aqueuse à 10% de bicarbonate de sodium. Une extraction à l'éther, suivie des traitements usuels de séparation, neutralisation, séchage et évaporation ont permis d'obtenir un résidu qui, par distillation, a fourni les produits désirés. L'analyse du distillat a été effectuée par chromatographie en phase gazeuse sur une colonne Carbowax (marque enregistrée) (10 m, capillalre): 130-170°/4° par min.

Une autre série d'essais a été effectuée en variant la proportion d'acide. A cet effet, on a procédé de la manière suivante.
Une solution constituée par 2,4 g (8,9 mmole) d'un mélange isomérique E/Z de l'alcool de départ (2:1) dans 5 ml de CH₂Cl₂ a été ajoutée goutte à goutte pendant 15 min à un mélange de l'acide dans 25 ml de CH₂Cl₂ à -50° sous azote. Le mélange réactionnel a été ensuite traité comme décrit ci-dessus. Les résultats obtenus sont résumés dans le tableau suivant.

**TABLEAU**

| Essai n° | Acide | Quantité acide [g] | Produits/Rendements (%) | | |
|---|---|---|---|---|---|
| | | | a | b | c |
| 22 | H₂SO₄ | 1,2 | 6 | 4 | 2 |
| 23 | H₂SO₄ | 2,4 | 24 | 19 | 7 |
| 24 | H₂SO₄ | 3,6 | 24 | 18 | 5 |
| 25 | H₂SO₄ | 4,8 | 27 | 23 | 10 |
| 26 | H₂SO₄ | 4,8 | 12 | 21 | 9 |
| 27 | H₂SO₄ | 7,2 | 19 | 20 | 8 |
| 28 | H₂SO₄ | 9,6 | 5 | 21 | 5 |
| 29 | H₂SO₄/oleum* | 2,6 | 22 | 17 | 6 |
| 30 | oleum | 2,4 | 18 | 14 | 5 |
| 31 | oleum | 4,8 | 18 | 15 | 5 |

| | | | | | |
|---|---|---|---|---|---|
| (*) 10:1 | | | | | |

Enfin, une série d'essais a été effectuée afin d'examiner l'influence de la proportion d'acide utilisée et de la température sur les rendements de la cyclisation et la nature des isomères obtenus. Les résultats obtenus sont résumés dans le tableau suivant. Le mode opératoire suivi est décrit ci-après.
Une solution à 30% de l'alcool de départ, mélange isomérique E/Z 2:1 (2,4 g; 8,9 mmole) dans le solvant choisi a été ajoutée goutte à goutte pendant 30 min à un mélange maintenu sous agitation de H₂SO₄ à 98%. Après un temps de réaction de 30 min à 1 h, pendant lequel le mélange réactionnel a été agité à la température indiquée, le mélange a été soumis aux traitements usuels décrits dans les essais précédents. Les résultats obtenus sont résumés dans le tableau ci-après.

**TABLEAU**

| Essai n° | Solvant [ml] | H₂SO₄ [g] | Temp. [°C] | Produits/Rendements (%) | | |
|---|---|---|---|---|---|---|
| | | | | a | b | c |
| 32 | EtNO₂(10) | 2,4 | -60/-20 | 17 | 23 | 6 |
| 33 | EtNO₂(10) | 4,8 | -60/-40 | 22 | 31 | 7 |
| 34 | EtNO₂(220) | 108 | -60/-40 | 22 | 33 | 8 |
| 35 | EtNO₂(340) | 108 | -60/-40 | 23 | 36 | 7 |
| 36 | EtNO₂(30) | 4,8 | 0 | 5 | 20 | 9 |
| 37 | EtNO₂(30) | 7,2 | -60 | 32 | 29 ¹⁾ | 4 |
| 38 | EtNO₂(30) | 7,2 | -60 | 1 | 68 ²⁾ | 9 |
| 39 | n-PrNO₂(30) | 4,8 | -40 | 21 | 28 | 6 |
| 40 | n-PrNO₂(30) | 7,2 | -60 | 22 | 34 | 6 |
| 41 | i-PrNO₂(30) | 2,4 | -50/20 | 11 | 13 | 5 |
| 42 | i-PrNO₂(30) | 7,2 | -60 | 19 | 36 | 6 |

| | | | | | | |
|---|---|---|---|---|---|---|
| 1) alcool (E) pur (97%) | | | | | | |
| 2) alcool (Z) pur (90%) | | | | | | |

Dans la majorité des conditions de réaction examinées, la formation d'isomères décaliniques trans est largement favorisée par rapport à celle de leurs isomères cis correspondants.

La cyclisation de 4-méthyl-6-(2,6,6-triméthyl-cyclohex-2-ényl)-hex-3-énol (alcool A) sous ses formes isomériques (E)- et (Z) a été effectuée sur 100 mg de l'alcool de départ dans un excès d'acide sulfurique à 98% (5 équiv. mol.) dans le dichlorométhane à -50° pendant 3h. Le mélange de réaction a été soumis aux traitements habituels tel que décrits dans les essais mentionnés plus haut.
Il en a été de même pour la cyclisation de (E)- et (Z)-4-méthyl-6-(2-méthylène-6,6-diméthyl-cyclohexyl)-hex-3-énol (alcool B). Le tableau suivant résume les résultats obtenus.

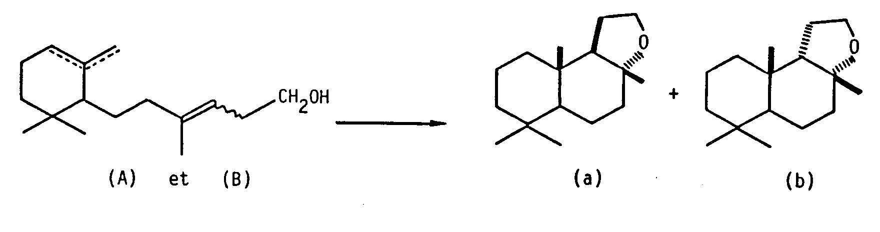

**TABLEAU**

| Alcool | Produits/Rendements (%) | |
|---|---|---|
| | a | b |
| (E)-A | 19 | 5 |
| (Z)-A | 2 | 27 |
| (E)-B | 25 | 6 |
| (Z)-B | 3 | 37 |

Les alcools utilisés comme produits de départ dans le procédé décrit plus haut peuvent être obtenus suivant la méthode que voici.
36 g (0,2 mole) de méthylate de sodium à 30% dans le méthanol ont été introduits goutte à goutte sous vigoureuse agitation dans un mélange constitué de 41,2 g (0,2 mole) de 2-méthyl-4-(2,6,6-triméthyl-cyclohex-1-ényl)-but-2-énal et 40 g (0,22 mole) de diméthylphosphonate de méthyle sous atmosphère d'azote. La réaction est exothermique. Après l'introduction, on a maintenu le mélange à reflux pendant 1 h. Après refroidissement, on a ajouté 30 ml d'eau et on a repris à l'éther de pétrole. La phase organique a été séparée puis lavée jusqu'à neutralité, séchée et concentrée. Par distillation sur une colonne Vigreux de 10 cm de longueur, on a obtenu 48,2 g de 4-méthyl-6-(2,6,6-triméthyl-cyclohex-1-ényl)-hexa-2,4-diénoate de méthyle.
Eb. 102°/4 Pa; rend. 91,5%.
Le 2-méthyl-4-(2,6,6-triméthyl-cyclohex-1-ényl)-but-2-énal est un produit commercial (origine: L. Givaudan).
L'ester obtenu suivant la méthode décrite plus haut a été ensuite réduit à l'aide d'aluminohydrure de lithium selon la méthode que voici.
42,8 g (0,163 mole) de 4-méthyl-6-(2,6,6-triméthyl-cyclohex-1-ényl)-hexa-2,4-diénoate de méthyle dilués avec un volume égal de THF ont été ajoutés goutte à goutte à une suspension de 6,2 g (0,163 mole) d'aluminohydrure de lithium dans 500 ml de THF anhydre sous atmosphère d'azote. La réaction est exothermique et a été maintenue en dessous de 30° par refroidissement à l'aide d'un bain d'eau glacée. Le mélange a été ensuite chauffé au reflux pendant 5 h, puis il a été refroidi à 10°, température à laquelle on a ajouté lentement goutte à goutte 6,2 ml d'eau, 6,2 ml de NaOH 2N et enfin 18,6 ml d'eau. Le tout a été agité encore 5 min. Il s'est formé ainsi un précipité blanc que l'on a filtré. Après concentration, le filtrat clair a été distillé sur une colonne Vigreux de 10 cm de longueur sous une pression de 3 Pa. On a ainsi obtenu les alcools désirés sous forme d'un mélange isomérique (E)/(Z); Eb. 76°/3 Pa, rend. 85%.
- IR :: 3300 cm⁻¹

### isomère (E):

- IR :: 3310, 1462, 1378, 1356, 1200, 1040 et 870 cm⁻¹;
- ¹H-RMN :: 1,00(6H,s); 1,41(2H,m); 1,58(2H,m); 1,61(3H,s); 1,70(3H,s); 1,91(2H,t,J=6,5); 2,06(4H,s); 2,30(2H,q,J=7); 3,63(2H,t,J=7); 5,16(1H,t,J=7) delta ppm;
- SM :: M⁺ = 236 ; m/e: 137(77), 121(11), 107(15), 95(100), 81(83), 69(32), 55(35).

### isomère (Z):

Eb. 105-110°/1 Pa
- IR :: 3320, 1470, 1442, 1370, 1358, 1200, 1040, 870 et 824 cm⁻¹;
- ¹H-RMN :: 1,02(6H,s); 1,22(2H,m); 1,37(2H,m); 1,64(3H,s); 1,78(3H,s); 1,91(2H,t,J=6); 2,06(4H,m); 2,31(2H,q,J=7); 3,63(2H,t,J=6); 5,11(1H,t,J=7) delta ppm;
- SM:: M⁺ = 236; m/e: 137(100), 121(9), 107(12), 95(81), 81(51), 69(19).

De manière analogue, on a préparé les alcools isomères dérivés du 2-méthyl-4-(2,6,6-triméthyl-cyclohex-2-ényl)-but-2-énal et du 2-méthyl-4-(2-méthylène-6,6-diméthyl-cyclohexyl)-but-2-énal, ces derniers composés pouvant être obtenus suivant les méthodes décrites par K.-H. Schulte-Elte et al. [Nouv. J. Chim. 1978, 2, 427-30] et I.M. Heilbron et al. [J. Chem. Soc. 1942, 727], respectivement.

### 4-méthyl-6-(2,6,6-triméthyl-cyclohex-2-ényl)-hex-3-énol:

### isomère (E): Eb. 160-170°/2 Pa

- IR :: 3300, 2900, 1440, 1380, 1360, 1040, 812 cm⁻¹;
- ¹H-RMN :: 0,87(3H,s); 0,92(3H,s); 1,12(1H,m); 1,30-1,65(4H); 1,65(3H,s); 1,68(3H,s); 1,96(2H,m); 2,05(2H,m); 2,28(2H,q,J=7); 3,61(2H,t,J=7); 5,13(1H,t,J=7); 5,28(1H,s) delta ppm;
- SM:: M⁺=236; m/e: 136(74), 121(56), 109(41), 95(26), 81(100), 69(26), 55(31), 41(64).

### isomère (Z): Eb. 160-170°/2 Pa

- IR :: 3320, 2900, 1442, 1380, 1360, 1042, 760 cm⁻¹;
- ¹H-RMN :: 0,88(3H,s); 0,96(3H,s); 1,14(1H,m); 1,30-1,60(4H); 1,71(3H,s); 1,74(3H,s); 1,96(2H,m); 2,07(2H,m); 2,28(2H,q,J=7); 3,62(2H,t,J=7); 5,09(1H,t,J=7); 5,31(1H,s) delta ppm;
- SM :: M⁺=236; m/e: 136(38), 121(31), 109(42), 95(21), 81(100), 69(25), 55(21), 41(62).

### 4-méthyl-6-(2-méthylène-6,6-diméthyl-cyclohexyl)-hex-3-énol:

### isomère (E): Eb. 160-170°/2 Pa

- IR :: 3300, 2900, 1640, 1440, 1380, 1360, 1042, 884, 630 cm⁻¹;
- ¹H-RMN:: 0,84(3H,s); 0,92(3H,s); 1,21(1H,m); 1,35-1,85(7H); 1,64(3H,s); 1,92-2,12(3H); 2,29(2H,q,J=7); 3,62(2H,t,J=7); 4,54(1H,s); 4,76(1H,s); 5,12(1H,t,J=7) delta ppm;
- SM:: M⁺=236; m/e: 221(25), 177(25), 121(26), 109(61), 95(38), 81(84), 69(61), 55(41), 41(100).

### isomère (Z): Eb. 160-170°/2 Pa

- IR :: 3300, 2900, 1640, 1440, 1380, 1362, 1042, 884, 630 cm⁻¹;
- ¹H-RMN :: 0,83(3H,s); 0,92(3H,s); 1,22(1H,m); 1,34-1,70(6H); 1,73(3H,s); 1,80-2,15(4H); 2,25(2H,m); 3,60(2H,t,J=7); 4,58(1H,s); 4,79(1H,s); 5,11(1H,t,J=7) delta ppm;
- SM :: M⁺=236; m/e: 221(25), 177(27), 121(29), 109(70), 95(42), 81(90), 69(60), 55(47), 41(100).

### Exemple 2

### Cyclisation acide de (E)- et (Z)-4-méthyl-6-(2,5,6,6-tétraméthyl-cyclohex-2-ényl)-hex-3-énol

La réaction s'effectue conformément au procédé décrit à l'Exemple 1 à l'aide d'acide sulfurique à 98% et à -60°. A cet effet, 0,7 g de l'alcool (E)- de départ ont été traités avec 1,4 g de H₂SO₄ dans 5 ml de nitroéthane. Après avoir été laissé sous agitation pendant 1 h à -60°, le mélange de réaction a été versé sur de la glace et le tout a été extrait à l'éther. Après les traitements usuels de neutralisation et concentration, on a distillé sur un appareil à boules à une température de 150°/20 Pa. On a ainsi obtenu 0,45 g de méthyl-AMBROX sous forme d'un mélange d'isomères dont les constituants principaux étaient le suivants:
3a alpha,5a alpha,7alpha,9a alpha,9b bêta-dodécahydro-3a,6,6,7,9a-pentaméthylnaphto[2,1-b]furanne:
   - ¹H-RMN :: 0,84(3H,d,J=7); 0,97(3H,s); 0,98(3H,s); 1,05(3H,s); 1,14(3H,s); 3,85(2H,m) delta ppm;
   - SM :: M⁺=250; m/e: 235(100), 151(29), 137(25), 123(30), 109(30), 97(77), 83(36), 67(33), 55(52), 43(55).
3a bêta,5a alpha,7alpha,9a alpha,9b bêta-dodécahydro-3a,6,6,7,9a-pentaméthylnaphto [2,1-b]furanne:
   - ¹H-RMN :: 0,79(3H,d,J=7); 0,92(3H,s); 0,98(3H,s); 1,09(3H,s); 1,35(3H,s); 3,76(2H,m) delta ppm;
   - SM :: M⁺=250; m/e: 235(100), 151(27), 137(28), 123(31), 109(31), 97(89), 81(42), 67(37), 55(50), 43(76).
3a bêta,5a alpha,7alpha,9a alpha,9b alpha-dodécahydro-3a,6,6,7,9a-pentaméthylnaphto [2,1-b]furanne:
   - ¹H-RMN :: 0,81(3H,J=7,d); 0,93(3H,s); 1,04(3H,s); 1,14(3H,s); 1,15(3H,s); 3,85(2H,m) delta ppm;
   - SM:: M⁺=250; m/e: 235(88), 151(22), 137(21), 121(25), 109(29), 97(100), 81(26), 67(30), 55(42), 43(42).
3a alpha,5a alpha,7alpha,9a alpha,9b alpha-dodécahydro-3a,6,6,7,9a-pentaméthylnaphto [2,1-b]furanne:
   - ¹H-RMN :: 0,76(3H,d,J=7); 0,94(3H,s); 0,98(3H,s); 1,02(3H,s); 1,055(3H,s); 3,77(2H,m) delta ppm;
   - SM :: M⁺=250; m/e: 235(89), 151(17), 135(14), 121(21), 109(26), 97(100), 81(41), 69(34), 55(43), 43(47).

Par le même procédé, on a effectué la cyclisation de l'alcool (Z) et obtenu le méthyl-AMBROX sous forme d'un mélange isomérique dont les constituants principaux étaient les suivants:
3a alpha,5a bêta,7alpha,9a alpha,9b bêta-dodécahydro-3a,6,6,7,9a-pentaméthylnaphto [2,1-b]furanne:
   - ¹H-RMN :: 0,67(3H,s); 0,81(3H,s); 0,845(3H,J=7,d); 0,90(3H,s); 1,09(3H,s); 3,86(2H,m) delta ppm;
   - SM:: M⁺=250; m/e: 235(100), 151(33), 137(42), 109(20), 97(47), 81(13), 67(14), 55(16), 43(21).
3a alpha,5a bêta,7alpha,9a alpha,9b alpha-dodécahydro-3a,6,6,7,9a-pentaméthylnaphto [2,1-b]furanne:
   - ¹H-RMN :: 0,66(3H,s); 0,84(3H,d,J=7); 0,91(3H,s); 1,07(3H,s); 1,37(3H,s); 3,81(2H,m) delta ppm;
   - SM :: M⁺=250 ; m/e: 235(47), 151(100), 137(53), 123(17), 109(22), 95(27), 81(17), 67(17), 55(17), 43(25).

Ces différents isomères ont été séparés par chromatographie en phase gazeuse. Le 4-méthyl-6-(2,5,6,6-tétraméthyl-cyclohex-2-ényl)-hex-3-énol, utilisé comme produit de départ dans le procédé décrit ci-dessus peut être préparé à partir de l'aldéhyde 2-méthyl-4-(2,5,6,6-tétraméthyl-cyclohex-2-ényl)-but-2-énal selon la méthode décrite aux exemples précédents. L'aldéhyde cité a été préparé à partir de cis-et trans-irone, suivant la méthode que voici.
5 G de trans-irone, 4 g de chloracétate d'éthyle et 20 ml d'éther absolu ont été traités sous atmosphère d'argon avec du tert-butylate de potassium, préparé à partir de 50 ml de tert-butanol et 4,3 g de potassium. La réaction a été effectuée à température ambiante. Après avoir été laissé réagir à cette température pendant une nuit, le mélange de réaction a été concentré sous vide et extrait ensuite avec de l'éther. Le résidu obtenu a été repris avec 10 ml de soude à 10% et 30 ml d'éthanol, puis il a été maintenu à reflux pendant 1 h. Après acidification et extraction à l'éther, on a réuni les extraits organiques qui, après avoir subi les traitements usuels, ont fourni, à Eb. 150°/10 Pa, 2,7 g du trans-aldéhyde désiré.
L'isomère cis- de ce même aldéhyde a été obtenu suivant la même méthode que celle indiquée ci-dessus, l'irone de départ étant de configuration cis.

Les différents isomères du méthyl-AMBROX, obtenus comme indiqué plus haut, sont des entités chimiques nouvelles dotées de qualités organoleptiques intéressantes à caractère ambré, voire boisé-ambré et, de ce fait, ils peuvent être employés en parfumerie.

### Exemple 3

### Cyclisation acide (E)- et (Z)-1,4-diméthyl-6-(2,6,6-triméthyl-cyclohex-1-ényl)-hex-3-énol

La réaction s'effectue conformément au procédé décrit à l'Exemple 1 à l'aide d'acide sulfurique à 95% et à -20°. A cet effet, une solution dans CH₂Cl₂ (10 ml) d'un mélange isomérique E/Z 1,5:1 (4,2 g, 0,017 mole) de l'alcool susmentionné a été traitée avec 4,3 g de H₂SO₄ (0,042 mole) dans 40 ml de CH₂Cl₂. Après avoir été laissé sous agitation pendant 3 h à -20°, le mélange de la réaction a été versé sur une solution aqueuse saturée de NaHCO₃ (100 ml). Les phases ont été séparées et la phase aqueuse extraite à l'éther. Les phases organiques combinées ont été séchées (Na₂SO₄) et concentrées, et l'huile résiduelle distillée pour fournir 4,07 g d'une huile incolore qui a été purifiée par chromatographie sur colonne [silica gel (350 g)], toluène/acétate d'éthyle 19:1, ensuite acétate d'éthyle) pour fournir un mélange isomérique (2,92 g ; rend. 77%) contenant comme composants 12-méthyl-AMBROX et 12-méthyl-épi-AMBROX. Le tableau suivant résume les résultats obtenus dans cet essai, ainsi que ceux obtenus lors des cyclisations, dans des conditions similaires, des alcools de départ de configuration (E)- ou (Z)-.

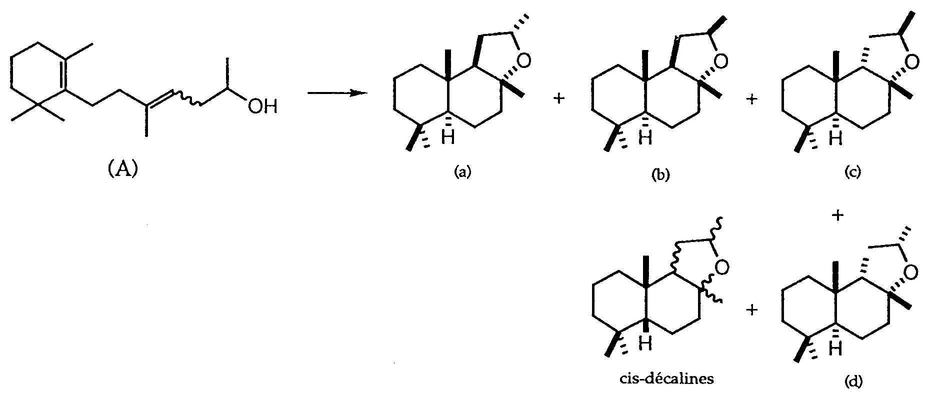

| Alcool | Produits/Rendements (%) | | | | |
|---|---|---|---|---|---|
| | a | b | c | d | cis-décalines |
| (E)-A | 25 | 20 | 8 | 4 | 11 |
| (Z)-A | 2 | 2 | 26 | 23 | 12 |
| (E/Z 1,5:1)-A | 17 | 15 | 19 | 12 | 12 |

### Données analytiques

**a** 2 bêta,3a alpha,5a bêta,9a alpha,9b bêta-dodécahydro-2,3a,6,6,9a-pentaméthylnaphto[2,1-b]furanne
   - RMN(¹H,360MHz):: 0,83(2s,6H); 0,88(s,3H); 1,11(s,3H); 1,19(d,J=7Hz,3H); 4,21(m,1H) delta ppm;
   - SM :: 250(0,5,M⁺), 235(100), 217(8), 151(10), 137(36), 111(52), 95(34), 81(41).
**b** 2 alpha,3a alpha,5a bêta,9a alpha,9b bêta-dodécahydro-2,3a,6,6,9a-pentaméthylnaphto[2,1-b]furanne
   - RMN(¹H,360MHz):: 0,83(s,3H); 0,85(s,3H); 0,87(s,3H); 1,14(s,3H); 1,29(d,J=7Hz,3H); 4,07(m,1H) delta ppm ;
   - SM :: 250(3,M⁺), 235(100), 217(8), 151(18), 137(37), 111(56), 95(36).
**c** 2 alpha,3a alpha,5a bêta,9a alpha,9b alpha-dodécahydro-2,3a,6,6,9a-pentaméthylnaphto[2,1-b]furanne
   - RMN(¹H,360MHz):: 0,81(s,3H); 0,89(s,3H); 1,09(s,3H); 1,16(d,J=7Hz,3H); 1,37(s,3H); 4,11(m,1H) delta ppm;
   - SM :: 250(6,M⁺), 235(85), 151(30), 137(100), 111(57), 95(58), 81(60), 43(98).
**d** 2 bêta,3a alpha,5a bêta,9a alpha,9b alpha-dodécahydro-2,3a,6,6,9a-pentaméthylnaphto[2,1-b]furanne
   - RMN(¹H,360MHz):: 0,81(s,3H); 0,89(s,3H); 1,09(s,3H); 1,26(d,J=7Hz,3H); 1,37(s,3H); 4,04(m,1H) delta ppm ;
   - SM :: 250(8,M⁺), 235(46), 151(46), 137(95), 109(52), 95(48), 81(58), 43(100).

Les alcools utilisés comme produits de départ dans le procédé décrit ci-dessus ont été obtenus à partir de 4-méthyl-6-(2,6,6-triméthyl-cyclohex-1-ényl)-hex-3-énol (voir Exemple 1) selon la méthode que voici [voir Swern, J. Org. Chem. (1978), 43, 2480].: Une solution de DMSO (2,2 g) dans CH₂Cl₂ (7 ml) a été ajoutée pendant 5 min à une solution maintenue sous agitation de chlorure d'oxalyle (1,8 g, 14,2 mmole) dans CH₂Cl₂ (30 ml) à -50°. Après 3 min, une solution de 4-méthyl-6-(2,6,6-triméthyl-cyclohex-1-ényl)-hex-3-énol (E/Z 1,5:1 ; 3 g, 12,7 mmole) dans CH₂Cl₂ (13 ml) a été ajoutée pendant 5 min et le mélange laissé sous agitation à -50° jusqu'à -30° pendant 2 h. Le mélange réactionnel a été traité avec (C₂H₅)₃N (6,5 g, 64,3 mmol) et la phase aqueuse extraite avec CH₂Cl₂. Le produit brut constitué par un mélange E/Z 1,5:1 de l'aldéhyde correspondant, présentant les caractéristiques analytiques suivantes, a été utilisé tel quel dans la poursuite de la synthèse.

### isomère (E):

- RMN(¹H,360MHz):: 3,14(d,J=7Hz,2H); 9,66(m,1H) delta ppm;
- SM :: 234(6,M⁺), 201(7), 178(9), 160(11), 145(18), 137(27), 123(77), 110(67), 95(99), 81(100).

### isomère (Z):

- RMN(¹H,360MHz):: 3,16(d,J=7Hz,2H); 9,66(m,1H) delta ppm;
- SM :: 234(2,M⁺), 201(2), 160(2), 145(5), 137(81), 121(16), 109(11), 95(100), 81(75).

Ce produit brut a été ensuite dissous dans (C₂H₅)₂O (10 ml) et la solution ajoutée goutte à goutte pendant 5 min à une solution fraîchement préparée de CH₃MgI (15 mmole) dans (C₂H₅)₂O (30 ml) à 0°- 10°. Après 1 h à température ambiante, le mélange a été versé sur NH₄Cl aqueux à 10% et extrait à l'éther. Le traitement usuel et la séparation chromatographique (silica gel) du mélange obtenu (2,2 g; E/Z 1,5:1) ont fourni les alcools désirés à l'état pur.

### (Z)-1,4-diméthyl-6-(2,6,6-triméthyl-cyclohex-1-ényl)-hex-3-énol

R_{f} 0,25 (toluene/acétate d'éthyle 9:1)
- IR:: 3530, 3390 (large), 1434, 1360, 1340, 1240, 1100, 1050, 1030, 920 cm⁻¹;
- RMN(¹H,360MHz,D₂O):: 1,02(s,6H); 1,20(d,J=7Hz,3H); 1,42(m,2H); 1,57(m,2H); 1,65(s,3H); 1,79(s,3H); 1,92(t,J=7Hz,2H); 1,96-2,12(4H); 2,18(m,2H); 3,79(m,1H); 5,14(t,J=7Hz,1H) delta ppm ;
- RMN(¹³C):: 139,6(s); 137,1(s); 127,3(s); 120,3(d); 68,0(d); 39,9(t); 38,0(t); 35,0(s); 32,9(t); 32,8(t); 28,7(2q); 27,3(t); 23,5(q); 22,9(q); 19,9(d); 19,6(t) delta ppm;
- SM :: 250(0,M⁺), 137(95), 121(17), 107(18), 95(100), 81(74), 69(28).

### (E)-1,4-diméthyl-6-(2,6,6-triméthyl-cyclohex-1-ényl)-hex-3-énol

R_{f} 0,21 (toluene/acétate d'éthyle 9:1)
- IR :: 3540, 3390 (large), 1440, 1370, 1350, 1250, 1110, 1036, 922 cm⁻¹;
- RMN(¹H,360MHz,D₂O):: 1,00(s,6H); 1,20(d,J=7Hz,3H); 1,42(m,2H); 1,57(m,2H); 1,61(s,3H); 1,69(s,3H); 1,91(t,J=7Hz,2H); 2,07(s,4H); 2,18(m,2H); 3,81(m,1H); 5,20(t,J=7Hz,1H) delta ppm;
- RMN(¹³C):: 139,8(s); 137,1(s); 127,1(s); 119,4(d); 68,0(d); 40,5(t); 39,9(t); 38,0(t); 35,0(s); 32,8(t); 28,7(2q); 28,0(t); 22,8(q); 19,8(q); 19,6(t); 16,4(q) delta ppm;
- SM:: 250(1,M⁺), 137(97), 121(19), 107(15), 95(100), 81(81), 69(25).

### Exemple 4

### Cyclisation acide de (E)- et (Z)-5-méthyl-7-(2,6,6-triméthyl-cyclohex-1-ényl)-hept-4-énol

La réaction s'effectue conformément au procédé décrit à l'Exemple 1, à l'aide d'acide sulfurique à 95% et à -20°, sous azote. A cet effet, une solution de (E)- ou (Z)-5-méthyl-7-(2,6,6-triméthyl-cyclohex-1-ényl)-hept-4-énol (2,1 g, 8,1 mmole) dans CH₂Cl₂ (5 ml) a été traitée avec 2,1 g de H₂SO₄ (0,02 mole) dans 21 ml de CH₂Cl₂. Après avoir été laissé sous agitation pendant 3 h, le mélange de la réaction a été versé sur NaHCO₃ à 10% (50 ml) et extrait à l'éther. Après les traitements usuels, on a distillé au four à boules et purifié l'huile obtenue par chromatographie sur colonne [silica gel (370 g), cyclohexane/acétate d'éthyle 7:3].
Le produit que l'on a ainsi obtenu contenait plusieurs isomères de l'ambra oxyde ou dodécahydro-4a,7,7,10a-tétraméthyl-1H-naphto[2,1-b]pyranne, ainsi que des produits secondaires non intéressants. Parmi lesdits isomères, on a identifié les structures suivantes :

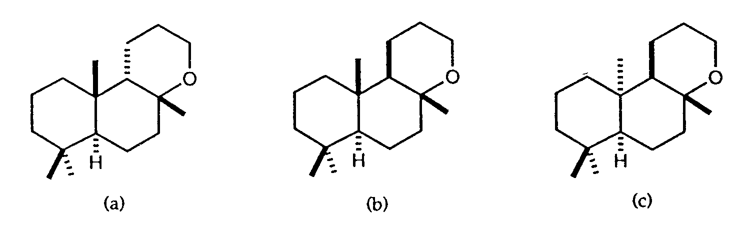

Deux autres composés, diastéréoisomères de (a) et (c), ont été détectés en faibles quantités et leur structure n'a pas pu être déterminée.
Le tableau ci-après résume les résultats obtenus :

| Alcool | Produits/Rendements (%) | | | |
|---|---|---|---|---|
| | a | b | c | autres isomères |
| (E) | 3 | 16 | 2 | 3 |
| (Z) | 6 | 25 | 2 | - |

### Données analytiques

**a** 4a alpha,6a bêta,10a alpha,10b alpha-dodécahydro-4a,7,7,10a-tétraméthyl-1H-naphto[2,1-b]pyranne
   - R_{f} (cyclohexane/acétate d'éthyle 7:3):: 0,60
   - RMN(¹H,360MHz):: 0,80(s,3H); 0,87(s,3H); 1,12(s,3H); 1,39(s,3H) delta ppm;
   - SM :: 250(15,M⁺), 235(55), 137(100), 111(92), 95(77), 81(76).
**b** 4a alpha,6a bêta,10a alpha,10b bêta-dodécahydro-4a,7,7,10a-tétraméthyl-1H-naphto[2,1-b]pyranne
   P.f. 81-83°
   - R_{f} (cyclohexane/acétate d'éthyle 19:1):: 0,32
   - IR:: 2920, 2850, 1446, 1380, 1364, 1120, 1078, 980 cm⁻¹;
   - RMN(¹H,360MHz):: 0,75(s,3H); 0,80(s,3H); 0,87(s,3H); 0,90-1,80(16H); 1,25(s,3H); 3,65(2H) delta ppm;
   - RMN(¹³C):: 74,6(s); 60,8(t); 57,8(d); 56,4(d); 42,1(t); 42,0(t); 39,0(t); 36,9(s); 33,3(q); 33,3(s); 27,7(t); 21,3(q); 19,9(q); 19,9(t); 18,6(t); 18,1(t); 15,5(q) delta ppm ;
   - SM :: 250(0,5,M⁺), 235(100), 137(32), 111(75), 95(43), 81(48), 69(40), 55(46), 43(55).
**c** 4a alpha,6a bêta,10a bêta,10b bêta-dodécahydro-4a,7,7,10a-tétraméthyl-1H-naphto[2,1-b]pyranne
   - R_{f} (cyclohexane/acétate d'éthyle 19:1):: 0,32
   - SM :: 250(1,M⁺), 235(100), 137(26), 121(27), 111(82), 95(42), 81(49).

Les alcools utilisés comme produits de départ dans le procédé décrit ci-dessus ont été préparés par une méthode alternative à celle représentée au schéma I, faisant appel à des réactions de type classique. Ils ont été obtenus à partir de la dihydro-bêta-ionone, comme suit.
Une solution de 4-(2,6,6-triméthyl-cyclohex-1-ényl)-butan-2-one (150 g, 0,77 mole) dans le tétrahydrofuranne (THF, 500 ml) a été ajoutée goutte à goutte, pendant 40 min, à une solution agitée de bromure de vinylmagnésium [0,93 mole; fraîchement préparée à partir de Mg (22,3 g, 0,93 mole) et bromure de vinyle (100 g, 0,93 mole)] dans le THF (1 l) à reflux et sous N₂. Une fois l'addition terminée, le mélange a été chauffé à reflux pendant 1 h et refroidi. On lui a alors ajouté soigneusement, goutte à goutte, NH₄Cl saturé (250 ml) et H₂O (300 ml). Les phases ont été séparées et la phase aqueuse a été extraite à l'éther. Après les traitements usuels de neutralisation, concentration et séchage des phases organiques combinées, on a soumis le produit à une distillation fractionnée pour obtenir 151 g de 3-méthyl-5-(2,6,6-triméthyl-cyclohex-1-ényl)-pent-1-én-3-ol sous forme d'une huile incolore.
P. éb. 85-88°/20 Pa; rend. 88%
- R_{f} (toluène/acétate d'éthyle 19:1):: 0,26
- IR :: 3410 (large), 1475, 1456, 1410, 1360, 1104, 998, 910 cm⁻¹;
- RMN(¹H,360MHz,D₂O):: 0,98(s,6H); 1,31(s,3H); 1,41(m,2H); 1,58(s,3H); 1,50-1,65(4H); 1,89(t,J=6,5Hz,2H); 2,01(m,2H); 5,08(d,J=11Hz,1H); 5,23(d,J=18Hz,1H), 5,95(dd,J=18, 11Hz,1H) delta ppm;
- SM :: 222(3,M⁺), 204(10), 189(31), 133(28), 121(54), 107(31), 95(100), 81(56), 71(33), 55(39), 41(48).

Un mélange de l'alcool préparé ci-dessus (25 g, 0,104 mole), orthoacétate de triméthyl (72 g, 0,6 mole) et acide propionique (0,4 g, 5,4 mmole) a été chauffé à reflux pendant 4 h et pendant que la température du bain était progressivement augmentée (de 135 à 150°) et que l'on distillait continuellement (colonne Vigreux 50 cm) les produits volatiles de la réaction [CH₃OH + CH₃C(OCH₃)₃]. Après 4 h, la température interne était de 116-117° et le mélange a été refroidi à température ambiante avant l'addition d'une deuxième portion d'acide propionique (0,4 g). Le mélange a été réchauffé à reflux pendant 20 h (température du bain d'huile: 140°; température interne: 117-119°) pendant que l'on éliminait de nouveau continuellement les composants volatiles. L'huile résiduelle a été distillée à pression atmosphérique pour enlever l'excès de CH₃C(OCH₃)₃ (impure: 46 g, p. éb. 108-110°/9,8x10⁴ Pa) et ensuite à pression réduite pour fournir 4 g de pentanol de départ non réagi (83% conversion) et 23,5 g de 5-méthyl-7-(2,6,6-triméthyl-cyclohex-1-ényl)-hept-4-énoate de méthyle (E/Z = 1,3:1) sous forme d'une huile incolore.
P. éb. 110-118°/6,7 Pa
- IR :: 2910, 1438, 1360, 1250, 1200, 1160, 986, 890 cm⁻¹

### isomère E

- R_{f} (toluène):: 0,31
- RMN(¹H,360MHz):: 0,99(s,6H); 1,42(m,2H); 1,55-1,65(2H); 1,60(s,3H); 1,66(s,3H); 1,90(t,J=7Hz,2H); 1,95-2,12(4H); 2,34(4H); 3,67(s,3H); 5,12(large t,J=7Hz,1H) delta ppm;
- SM:: 278(0,5,M⁺), 137(99), 121(13), 109(15), 95(100), 81(87), 67(26), 55(21).

### isomère Z

- R_{f} (toluène):: 0,35
- RMN(¹H,360MHz):: 1,02(s,6H); 1,42(m,2H); 1,55-1,65(2H); 1,65(s,3H); 1,74(s,3H); 1,92(t,J=7Hz,2H); 1,95-2,12(4H); 2,34(4H); 3,67(s,3H); 5,07(large t,J=7Hz,1H) delta ppm;
- SM :: 278(1,M⁺), 137(96), 121(13), 109(12), 95(100), 81(87), 67(28), 55(23).

Une solution du mélange isomérique (E/Z = 1,3:1, 18 g, 0,063 mole) de l'ester obtenu ci-dessus dans l'éther (100 ml) a été ajoutée goutte à goutte, pendant 15 min, à une pâte de LiAlH₄ (3,2 g, 0,084 mole) dans l'éther (100 ml) à reflux sous N₂. Au mélange refroidi, on a ajouté successivement H₂O (3,2 ml), NaOH à 20% (3,2 ml) et H₂O (10 ml). Après filtration, concentration du filtrat et distillation, on a obtenu 15,5 g (rend. 98%) de 5-méthyl-7-(2,6,6-triméthyl-cyclohex-1-ényl)-hept-4-énol (E/Z = 1,3:1, p. éb. 113-123°/6,7 Pa). Les deux isomères ont été obtenus à l'état pur par chromatographie sur colonne de 12 g du mélange isomérique [silica gel (2x800 g), toluène/acétate d'éthyle 9:1).

### (E)-5-méthyl-7-(2,6,6-triméthyl-cyclohex-1-ényl)-hept-4-énol

- IR :: 3320(large), 1440, 1380, 1360, 1200, 1060, 878 cm⁻¹ ;
- RMN(¹H,360MHz,D₂O):: 1,00(s,6H); 1,41(m,2H); 1,57(m,2H); 1,60(s,3H); 1,63(m,2H); 1,66(s,3H); 1,91(t,J=6Hz,2H); 1,97-2,13(6H); 3,65(t,J=7Hz,2H); 5,17(t,J=7Hz, 1H) delta ppm;
- RMN(¹³C):: 137,2(s); 136,9(s); 127,0(s); 123,2(d); 62,7(t); 40,3(t); 39,9(t); 35,0(s); 32,8(t); 28,7(2q); 28,0(t); 24,3(t); 19,8(q); 19,6(t); 16,0(q) delta ppm ;
- SM :: 250(2,M⁺), 137(76), 121(25), 95(100), 81(74), 67(31), 55(30).

### (Z)-5-méthyl-7-(2,6,6-triméthyl-cyclohex-1-ényl)-hept-4-énol

- IR :: 3320(large), 1448, 1380, 1360, 1200, 1060 cm⁻¹ ;
- RMN(¹H,360MHz,D₂O):: 1,02(s,6H); 1,42(m,2H); 1,57(m,2H); 1,62(m,2H); 1,64(s,3H); 1,74(d,J=1,5Hz,3H); 1,92(large t,J=6Hz,2H); 1,97-2,14(6H); 3,64(t,J=7Hz,2H); 5,12(t,J=7Hz,1H) delta ppm;
- RMN(¹³C):: 137,2(s); 136,6(s); 127,1(s); 124,2(d); 62,6(t); 40,0(t); 35,0(s); 33,2(t); 32,9(t); 32,7(2t); 28,7(2q); 27,4(t); 24,4(t); 23,3(q); 19,9(q); 19,6(t) delta ppm
- SM :: 250(2,M⁺), 137(78), 121(23), 95(100), 81(81), 67(29), 55(30).

## Revendications

1. Procédé pour la préparation d'éthers polycycliques de formule 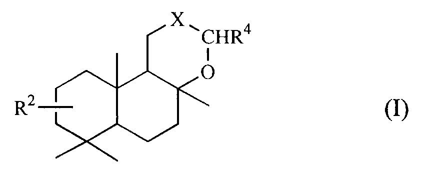 dans laquelle X représente un groupe ―(CH₂)ₙ―, l'indice n définit un nombre entier de valeur 0 ou 1, le symbole R² représente un atome d'hydrogène ou un radical alkyle inférieur de C₁ à C₃ et le symbole R⁴ représente un atome d'hydrogène ou un radical méthyle, caractérisé en ce qu'on cyclise à l'aide d'un agent acide un composé polyinsaturé de formule 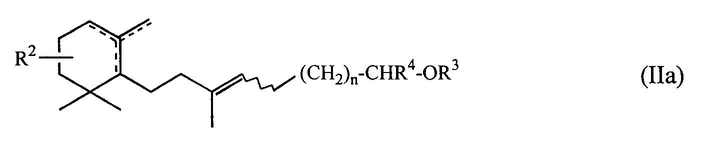 possédant une double liaison dans l'une des positions indiquées par les pointillés et dans laquelle les symboles n, R² et R⁴ sont définis comme à la formule (I), la ligne ondulée représente une liaison C-C de configuration cis ou trans et le symbole R³ représente un atome d'hydrogène ou un groupe protecteur de la fonction hydroxyle lié à l'atome d'oxygène et pouvant se dissocier de celui-ci dans les conditions de la réaction.

2. Procédé selon la revendication 1, caractérisé en ce qu'on cyclise un composé polyinsaturé de formule 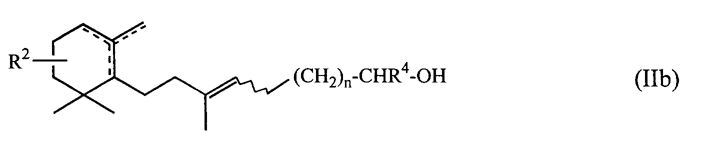 possédant une double liaison dans une des positions indiquées par les pointillés, et dans laquelle la ligne ondulée représente une liaison C-C de configuration cis ou trans et les symboles n, R² et R⁴ sont définis comme à la revendication 1.

3. Procédé selon la revendication 2, caractérisé en ce qu'on cyclise un composé polyinsaturé de formule (IIb) dans laquelle R² représente un atome d'hydrogène ou un radical méthyle, pour obtenir un composé de formule 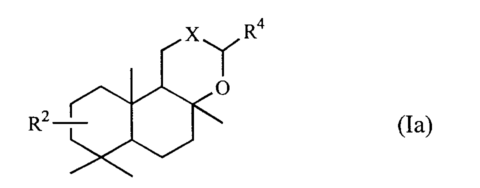 dans laquelle le symbole R² a le sens indiqué ci-dessus et les symboles X et R⁴ sont définis comme à la revendication 1.

4. Procédé selon la revendication 1, caractérisé en ce qu'on cyclise un composé polyinsaturé de formule 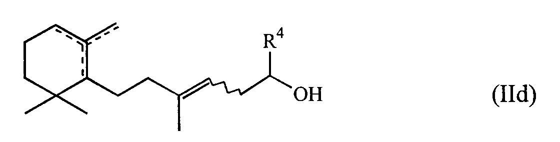 possédant une double liaison dans l'une des positions indiquées par les pointillés et dans laquelle la ligne ondulée représente une liaison C-C de configuration cis ou trans et le symbole R⁴ représente un atome d'hydrogène ou un radical méthyle, pour obtenir un composé de formule 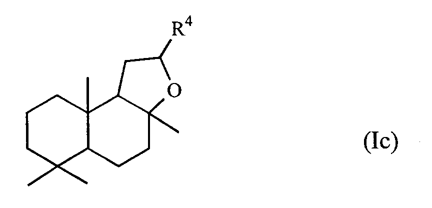 dans laquelle le symbole R⁴ représente un atome d'hydrogène ou un radical méthyle.

5. Procédé selon la revendication 4, caractérisé en ce qu'on cyclise un composé polyinsaturé de formule (IId) dans laquelle la ligne ondulée définit une liaison C-C de configuration trans et le symbole R⁴ représente l'hydrogène, pour fournir un mélange isomérique de l'éther (Ic) correspondant contenant une quantité prépondérante de l'isomère de formule 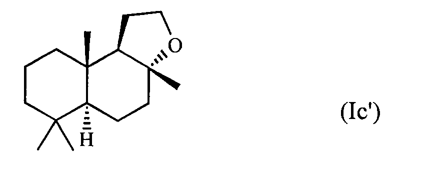 ou 3aα,5aβ,9aα,9bβ-dodécahydro-3a,6,6,9a-tétraméthyl-naphto[2,1-b]furanne.

6. Procédé selon la revendication 4, caractérisé en ce qu'on cyclise un composé polyinsaturé de formule (IId) dans laquelle la ligne ondulée définit une liaison C-C de configuration cis et le symbole R⁴ représente l'hydrogène, pour fournir un mélange isomérique de l'éther (Ic) correspondant contenant une quantité prépondérante de l'isomère de formule 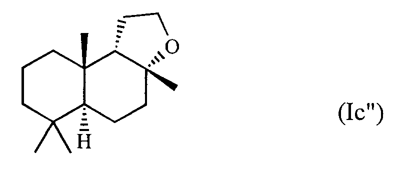 ou 3aα,5aβ,9aα,9bα-dodécahydro-3a,6,6,9a-tétraméthyl-naphto[2,1-b]furanne.

7. Composé polyinsaturé de formule 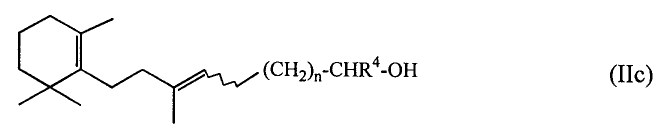 dans laquelle l'indice n définit un nombre entier de valeur 0 ou 1, le symbole R⁴ représente un atome d'hydrogène ou un radical méthyle et la ligne ondulée représente une liaison C-C de configuration cis ou trans, à l'exclusion du 4-méthyl-6-(2,6,6-triméthyl-cyclohéx-1-ényl)-hex-3-énol.

## Claims

1. Process for the preparation of polycyclic ethers of formula 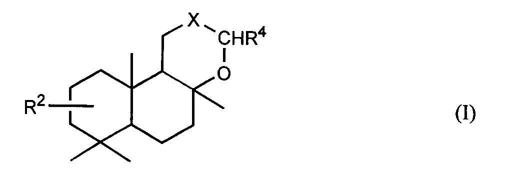 wherein X represents a -(CH₂)ₙ- group, index n stands for integer 0 or 1, symbol R² represents a hydrogen atom or a lower alkyl radical from C, to C₃ and symbol R⁴ represents a hydrogen atom or a methyl radical, which process is characterized by the cyclization by means of an acidic agent of polyunsaturated compound of formula 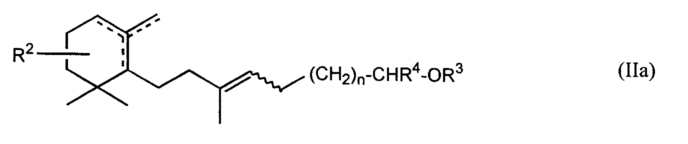 having a double bond in one of the positions indicated by the dotted lines, and wherein symbols n, R² and R⁴ are defined as in formula (I), the wavy line represents a C-C bond of cis or trans configuration and symbol R³ represents a hydrogen atom or a protecting group of the hydroxyl function bound to the oxygen atom and able to dissociate itself from the latter under the reaction conditions.

2. Process according to claim 1, characterized in that the cyclization is carried out on a compound of formula 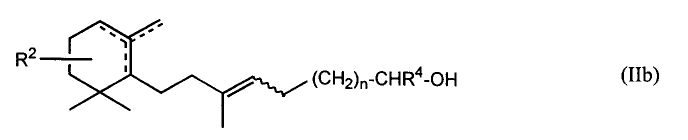 having a double bond in one of the positions indicated by the dotted lines and wherein the wavy line represents a C-C bond of cis or trans configuration and symbols n, R² and R⁴ are defined as in claim 1.

3. Process according to claim 2, characterized in that there is cyclized a polyunsaturated compound of formula (IIb) wherein R² represents a hydrogen atom or a methyl radical to provide a compound of formula 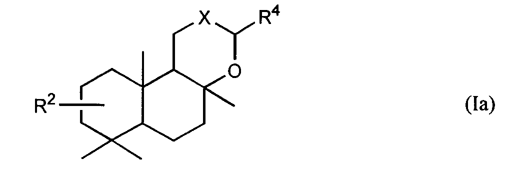 wherein symbol R² has the meaning indicated above and symbols X and R⁴ are defined as in claim 1.

4. Process according to claim 1, characterized in that there is cyclized a polyunsaturated compound of formula 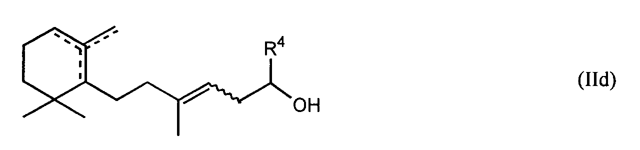 having a double bond in one of the positions indicated by the dotted lines and wherein the wavy line represents a C-C bond of cis or trans configuration and symbol R⁴ represents a hydrogen atom or a methyl radical, to provide a compound of formula 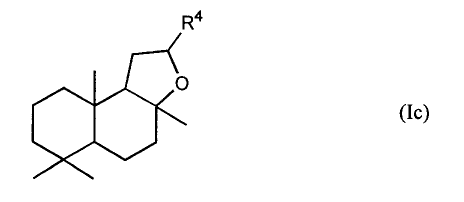 wherein symbol R⁴ represents a hydrogen atom or a methyl radical.

5. Process according to claim 4, characterized in that there is cyclized a polyunsaturated compound of formula (IId) wherein the wavy line represents a C-C bond of cis or trans configuration and symbol R⁴ represents a hydrogen atom, to provide an isomer mixture of corresponding ether (Ic) having a predominant amount of the isomer of formula 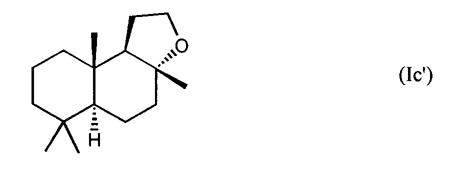 or 3aα,5aβ,9aα,9bβ-dodecahydro-3a,6,6,9a-tetramethyl-naphtho[2,1-b]furan.

6. Process according to claim 4, characterized in that there is cyclized a polyunsaturated compound of formula (IId), wherein the wavy line defines a C-C bond of cis or trans configuration and symbol R⁴ represents hydrogen, to provide an isomer mixture of the corresponding ether (Ic) having a predominant amount of the isomer of formula 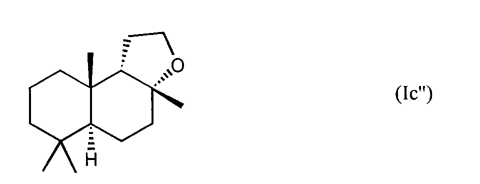 or 3aα,5aβ,9aα,9bβ-dodecahydro-3a,6,6,9a-tetramethyl-naphtho[2,1-b]furan.

7. Polyunsaturated compound of formula 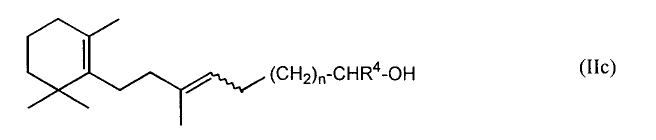 wherein index n defines an integer of 0 or 1, symbol R⁴ represents a hydrogen atom or a methyl radical and the wavy line represents a C-C bond of cis or trans configuration, provided that 4-methyl-6-(2,6,6-trimethyl-cyclohex-1-enyl)-hex-3-enol is excluded.

## Patentansprüche

1. Verfahren zur Herstellung von polycyclischen Ethern der Formel 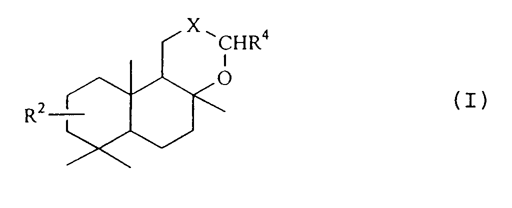 worin X für eine Gruppe -(CH₂)ₙ- steht, n eine ganze Zahl vom Wert 0 oder 1 bedeutet, das Symbol R² für ein Wasserstoffatom oder einen niederen C₁-C₃-Alkylrest steht und das Symbol R⁴ ein Wasserstoffatom oder einen Methylrest bedeutet, dadurch gekennzeichnet, dass man eine mehrfach ungesättigte Verbindung der Formel 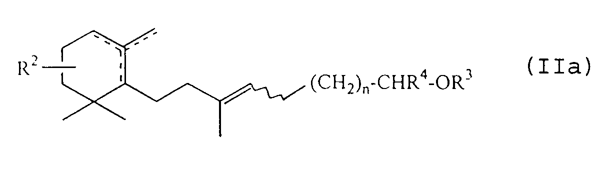 welche eine Doppelbindung in einer der durch die Punktierungen angegebenen Stellungen besitzt und worin die Symbole n, R² und R⁴ die in der Formel (I) angegebene Bedeutung besitzen, die gewellte Linie eine C-C-Bindung mit cis- oder trans-Konfiguration und das Symbol R³ ein Wasserstoffatom oder eine Schutzgruppe des Hydroxylrestes, welche an das Sauerstoffatom gebunden ist und sich von diesem unter den Reaktionsbedingungen abspalten kann, bedeuten, mit Hilfe eines sauren Mittels cyclisiert.

2. Verfahren gemäss Patentanspruch 1, dadurch gekennzeichnet, dass man eine mehrfach ungesättigte Verbindung der Formel 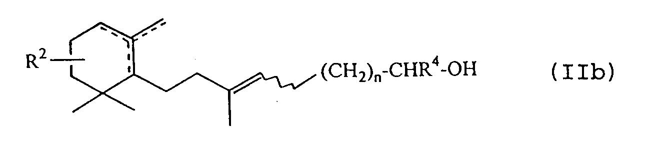 welche eine Doppelbindung in einer der durch die Punktierungen angegebenen Stellungen besitzt und worin die gewellte Linie eine C-C-Bindung mit cis- oder trans-Konfiguration bedeutet und die Symbole n, R² und R⁴ die in Anspruch 1 angegebene Bedeutung besitzen, cyclisiert.

3. Verfahren gemäss Patentanspruch 2, dadurch gekennzeichnet, dass man eine mehrfach ungesättigte Verbindung der Formel (IIb), worin R² für ein Wasserstoffatom oder einen Methylrest steht, cyclisiert, um eine Verbindung der Formel 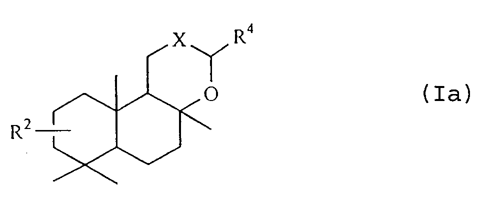 zu erhalten, worin das Symbol R² die oben angegebene Bedeutung besitzt und die Symbole X und R⁴ die im Patentanspruch 1 angegebene Bedeutung besitzen.

4. Verfahren gemäss Patentanspruch 1, dadurch gekennzeichnet, dass man eine mehrfach ungesättigte Verbindung der Formel 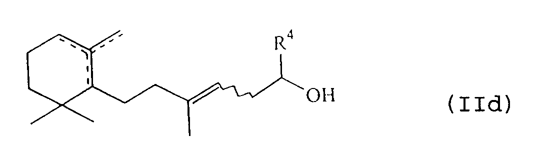 welche eine Doppelbindung in einer der durch die Punktierungen angegebenen Stellungen besitzt und worin die gewellte Linie eine C-C-Bindung mit cis- oder trans-Konfiguration bedeutet und das Symbol R⁴ für ein Wasserstoffatom oder einen Methylrest steht, cyclisiert, um eine Verbindung der Formel 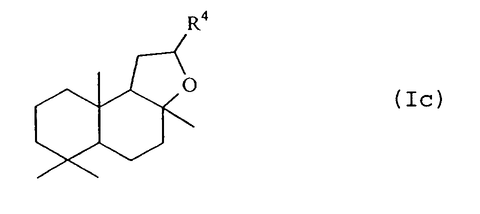 zu erhalten, worin das Symbol R⁴ ein Wasserstoffatom oder einen Methylrest bedeutet.

5. Verfahren gemäss Patentanspruch 4, dadurch gekennzeichnet, dass man eine mehrfach ungesättigte Verbindung der Formel (IId), worin die gewellte Linie eine C-C-Bindung mit trans-Konfiguration bedeutet und das Symbol R⁴ für Wasserstoff steht, cyclisiert, um ein Isomerengemisch des entsprechenden Ethers (Ic) zu ergeben, welches einen überwiegenden Anteil des Isomeren der Formel 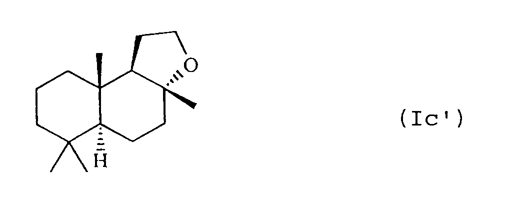 oder 3aα,5aβ,9aα,9bβ-Dodecahydro-3a,6,6,9a-tetramethyl-naphto[2,1-b]furan enthält.

6. Verfahren gemäss Patentanspruch 4, dadurch gekennzeichnet, dass man eine mehrfach ungesättigte Verbindung der Formel (IId), worin die gewellte Linie eine C-C-Bindung mit cis-Konfiguration bedeutet und das Symbol R⁴ für Wasserstoff steht, cyclisiert, um ein Isomerengemisch des entsprechenden Ethers (Ic) zu ergeben, welches einen überwiegenden Anteil des Isomeren der Formel 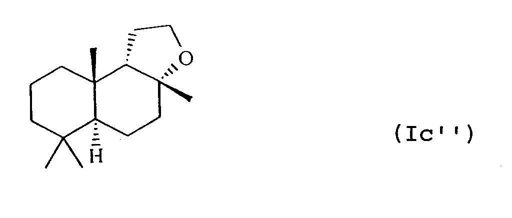 oder 3aα,5aβ,9aα,9bα-Dodecahydro-3a,6,6,9a-tetramethyl-naphto[2,1-b]furan enthält.

7. Mehrfach ungesättigte Verbindung der Formel 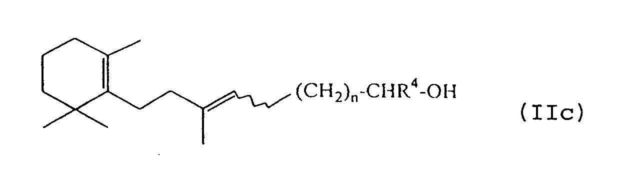 worin n eine ganze Zahl vom Wert 0 oder 1 bedeutet, das Symbol R⁴ für ein Wasserstoffatom oder einen Methylrest steht und die gewellte Linie eine C-C-Bindung mit cis-oder trans-Konfiguration bedeutet, mit Ausnahme von 4-Methyl-6-(2,6,6-trimethyl-cyclohex-1-enyl)-hex-3-enol.
